Europäisches Patentamt

European Patent Office

Office européen des brevets

(11) Veröffentlichungsnummer: **0 397 931**
A1

# EUROPÄISCHE PATENTANMELDUNG

(21) Anmeldenummer: 89202315.1

(22) Anmeldetag: 14.09.89

(51) Int. Cl.5: **A61F 5/02, A61F 5/04, A61H 23/04**

(30) Priorität: 21.04.89 EP 89107206

(43) Veröffentlichungstag der Anmeldung:
22.11.90 Patentblatt 90/47

(84) Benannte Vertragsstaaten:
AT BE CH DE FR GB IT LI LU NL SE

(71) Anmelder: **Pink, Nikolaus**
**Gasenbergstrasse 53**
**D-4600 Dortmund 30(DE)**

(72) Erfinder: **Pink, Nikolaus**
**Gasenbergstrasse 53**
**D-4600 Dortmund 30(DE)**

(54) Volumenregulator für medizinische Behandlungen.

(57) Der Volumenregulator bietet die Möglichkeit, während einer Behandlung Druckausübungen auf Körperteile zu steuern und Stützungsfunktionen konstant zu halten, wodurch sich Behandlungszeiten abkürzen lassen können oder weitere Behandlungsmöglichkeiten eröffnen.

EP 0 397 931 A1

**Beschreibung des Volumenregulators für medizinische Behandlungen**

Bei längerfristig erforderlichem "Eingipsen" oder anderweitigem Stützen von Körpern oder Körperteilen müßte die Paßform bei Gewichtsverlust oder -Zunahme jeweils erneuert werden.

Durch einen regulierbaren Luftdruck kann das Volumen jederzeit verändert bezw. angepaßt werden, wie auch ein gewünschter Andruck durch nachpumpen erhöht oder Luftablass vermindert werden.kann.

Kombinationen verschiedener Volumenregulatoren sind einfach herzustellen und miteiander zu verbinden oder in festgelegten Abständen zu fixieren.

Wechsel einzelner Volumenregulatoren aus medizinischen Erfordernissen oder hygienischen Gründen sind durch Ablassen der Luft einfach auszuführen.

Durch Kombination mit Pressluftflaschen und motorisch gesteuerten Ventilen können programmierte Situationen z.B. bei Bewußtlosen durchgeführt werden.

Das "Eingipsen" o.ä. könnte durch einen zylinderförmigen Körper aus Kunststoff oder Metall ersetzt werden. In der Orthopädie könnten mit diesem System Bewegungs-und Haltungskorrekturen durchgeführt werden.

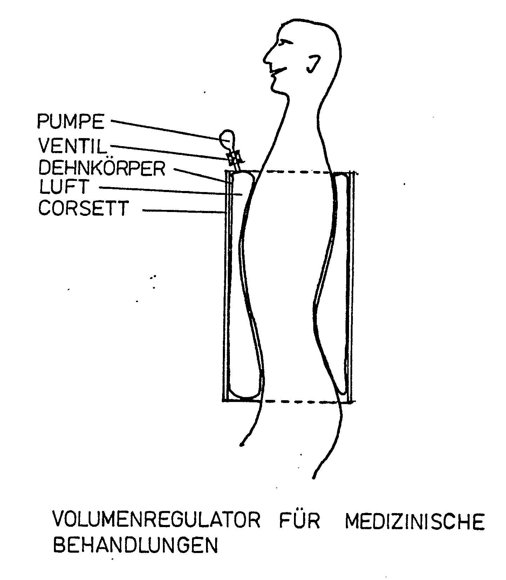

**Ansprüche**

1. Volumenregulator gebildet aus Dehnkörper, Ventil und Pumpe.

2. Dadurch gekennzeichnet, daß zwischen einem Widerlager und dem zu behandelndem Körper das Volumen veränderbar bezw. regulierbar ist.

3. Dadurch gekennzeichnet, daß der Dehnkörper auch aus Kombinationen unterschiedlich elastischer oder starrer Stoffe gebildet werden kann, um gewünschte Verformungen zu erzielen oder bestimmte Auswirkungen auf die zu behandelnden Körper zu bewirken.

PUMPE  
VENTIL  
DEHNKÖRPER  
LUFT  
CORSETT

VOLUMENREGULATOR FÜR MEDIZINISCHE BEHANDLUNGEN

Europäisches
Patentamt

**EUROPÄISCHER RECHERCHENBERICHT**

Nummer der Anmeldung

EP 89 20 2315

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-3 580 248 (LARSON) <br> * Figuren 1-4; Anspruch 1; Spalte 3, Zeile 58 - Spalte 4, Zeile 3 * <br> --- | 1 | A 61 F 5/02 <br> A 61 F 5/04 <br> A 61 H 23/04 |
| X | DE-A-1 566 367 (ZUMAGLINI) <br> * Figuren 1,3; Ansprüche 1,3,4,10 * <br> --- | 1 | |
| X | DE-A-2 913 606 (JOHNSON) <br> * Figuren 1,2; Ansprüche 1,2 * <br> --- | 1 | |
| X | FR-A-2 560 518 (BENABID) <br> * Figuren 6-11; Anspruch 1; Seite 3, Zeilen 23-32 * <br> --- | 1 | |
| A | US-A-2 781 041 (WEINBERG) <br> * Figur 1; Anspruch 2 * <br> ----- | 1 | |

**RECHERCHIERTE SACHGEBIETE (Int. Cl.5)**

A 61 F
A 61 H

Der vorliegende Recherchenbericht wurde für alle Patentansprüche erstellt

| Recherchenort | Abschlußdatum der Recherche | Prüfer |
|---|---|---|
| BERLIN | 24-07-1990 | KANAL P K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)